(19) Europäisches Patentamt
European Patent Office
Office européen des brevets

(11) Publication number: **0 121 907**
**B1**

(12) **EUROPEAN PATENT SPECIFICATION**

(45) Date of publication of patent specification: **23.07.86**

(51) Int. Cl.$^4$: **C 07 C 93/04**

(21) Application number: **84103797.1**

(22) Date of filing: **05.04.84**

# (54) Method for producing bis(beta-(N,N-dimethylamino)ethyl)ether.

(30) Priority: **07.04.83 JP 60076/83**

(43) Date of publication of application:
**17.10.84 Bulletin 84/42**

(45) Publication of the grant of the patent:
**23.07.86 Bulletin 86/30**

(84) Designated Contracting States:
**DE FR GB IT NL SE**

(56) References cited:
**US-A-3 426 072**

(73) Proprietor: **TOYO SODA MANUFACTURING CO., LTD.**
**No. 4560, Ooaza Tonda Shinnanyo-shi**
**Yamaguchi-ken (JP)**

(72) Inventor: **Kumoi, Sadakatsu**
**No. 6-7, Nijigaoka 5-chome**
**Hikari-shi Yamaguchi-ken (JP)**
Inventor: **Mitarai, Keiji**
**No. 1083, Ooaza Tonda**
**Shinnanyo-shi Yamaguchi-ken (JP)**
Inventor: **Tsutsumi, Yukihiro**
**No. 1723, Ooaza Kamishimo**
**Tokuyama-shi Yamaguchi-ken (JP)**

(74) Representative: **Patentanwälte TER MEER - MÜLLER - STEINMEISTER**
**Mauerkircherstrasse 45**
**D-8000 München 80 (DE)**

**EP 0 121 907 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Detailed description of the invention

The present invention concerns an improved method for producing bis[β - (N,N - dimethylamino)ethyl]ether.

It is known that bis[β - (N,N - dimethylamino)ethyl]ether is a compound of industrial usefulness as a catalyst for producing a polyurethane.

There has been generally known a method of producing a tertiary amine compound by reacting various kinds of neucleophilic reagents with a quarternary ammonium salt compound to effect a dealkylation reaction (14 III 1398 of New Experimental Chemistry Lecture, 1978). Likewise, a method for producing a corresponding bis[β - (N,N - dimethylamino)ethyl]ether (hereinafter abbreviated as "etheramine") by demethylation reaction of a bismethohalide of bis[β - (N,N - dimethylamino)ethyl]ether (hereinafter abbreviated as "bismethohalide") is disclosed in U.S.P. 3,400,157 and Japanese examined publication No. Sho 48-7411 (U.S.P. 3,426,072). In U.S.P. 3,400,157, a bismethochloride aqueous solution of bis[β - (N,N - dimethylamino)ethyl]-ether obtained by a reaction of dichloroethylether with 40% trimethylamine aqueous solution undergoes a demethylation reaction to affordsaid corresponding etheramine in a 67% yield, but it cannot be said to be fully high from aspect of yield.

In Japanese Publication No. Sho 48-7411 (U.S.P. 3,426,072), 71% aqueous solution of said bismethochloride and polyamine having boiling point of more than 220°C such as aminoethylethanolamine, triethylenetetramine and the like undergo a reaction at ordinary pressure or reduced pressure to afford the etheramine in 71% of the highest yield.

The method employing these polyamines of high boiling point finds some improvements in operations for separating and recovering the etheramine from the reaction mixture, so it can be said to be a comparatively excellent producing process from aspect of the operations, but it can not yet find full satisfaction in aspect of yield thereof. In order that this process employing dihaloethylether as the starting material which is comparatively expensive, in particular, may become an industrial process having excellent in economy, further improvements in aspect of yield is strongly desired.

Upon reviewing these circumstances, the inventors of this invention have made researches in the problem with their earnest efforts and found a new fact that the etheramine can be produced in a surprisingly high yield by selecting a reaction mode of heterogeneous system consisting of a solid-liquid mixed phase in which the reaction temperature and the quantity of water in the reaction mixture are controlled to particular conditions at the time of reacting the bismethohalide with an amine compound having boiling point of not lower than 220°C at the atmospheric pressure and having a primary amino group.

This invention provides a method for producing bis[β - (N,N - dimethylamino)ethyl]ether wherein an aqueous solution of a bismethohalide compound of bis[β - (N,N - dimethylamino)-ethyl]ether represented by the following general formula:

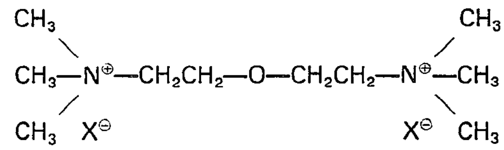

(X denotes a halogen atom)

is reacted with an aliphatic amine having boiling point of not lower than 220°C and having a primary amino group in the molecule, at atmospheric pressure, which is characterized in comprising two steps of (a) distilling off water at a temperature of not more than 130°C from the reaction mixture of said bismethohalide aqueous solution and said aliphatic amine to render the concentration of said bismethohalide to water to be not lower than 85% by weight, and (b) performing a heterogeneous reaction in the resulting solid-liquid phase mix by heating it at a temperature of now lower than 140°C. The starting bismethohalide used in this invention is represented by the following general formula:

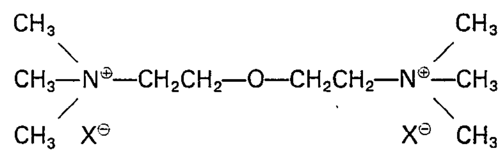

(X denotes a halogen atom) and, generally speaking, a bismethohalide of bis[β - (N,N - dimethylamino)ethyl]ether in which X denotes chlorine, bromine or the like, that is, the bismethochloride of bis [β - (N,N - dimethylamino)-ethyl]ether or the bismethobromide of bis[β - (N,N - dimethylamino)ethyl]ether can be widely used as the starting material, on account of economical standpoint, convenience for obtaining the starting material and so on.

These bismethohalides can be obtained as a homogeneous aqueous solution thereof by reacting dihaloethylether which are industrially obtainable with trimethylamine aqueous solution. The solubility of the bismethohalide into water varies the temperature, but is nearly about 71% by weight in a temperature range of 20—100°C, and so a homogeneous aqueous solution of the bismethohalide of below the above concentration is usually available. In other words, a homogeneous aqueous solution of the bismethohalide is used as the starting material, which is obtained by reacting dihaloethylether with trimethylamine aqueous solution.

The aliphatic amine employed in this invention is an aliphatic amine compound having a boiling

point of not below 220°C at the atmospheric pressure and having a primary amino group in the molecule thereof. These aliphatic amines contain a secondary amino group, a tertiary amino group, oxy group or hydroxy group in the molecule thereof, besides the primary amino group which gives rise to no particular restriction.

As typical aliphatic amines, there are exemplified polyethylenepolyamines such as triethylenetetramine, tetraethylenepentamine, pentaethylenehexamine and the like (these polyethylenepolyamines usually consist in a mixture of several kinds of ethyleneamines containing piperazine ring); polypropylenepolyamines such as dipropylenetriamine, tripropylenetetramine, tetrapropylenepentamine and the like; high boiling point polyamines such as N,N' - bis(3 - aminopropyl)ethylenediamino, N - aminoethylethanolamine, N - aminoethylpropanolamine and the like.

The reaction process in this invention comprises a reaction of two steps; first step of heating the reaction mixture of the starting bismethohalide aqueous solution and the high boiling point aliphatic amine at a temperature of not above 130°C, preferably not above 120°C, thereby distilling and removing water out of the reaction mixture under the ordinary pressure or reduced pressure to concentrate it up to a concentration of the bismethohalide to water being not below 85% by weight, preferably not below 90% by weight; second step of heating the resultant heterogeneous reaction mixture, in which a part of the bismethohalide deposits and disperses in the high boiling point aliphatic amine, at a temperature of not below 140°C, preferably not below 160°C to afford the resultant etheramine. The addition amount of the high boiling point aliphatic amine is usually not lower than 2 mols to 1 mol of the bismethohalide. Since the high boiling point aliphatic amine functions also as a dispersing agent of the bismethohalide, its dispersing property is not improved, which causes drop of the yield when not at least 2 mols, preferably not less than 3 moles were added. The upper limit of addition amount of the high boiling point aliphatic amine is not restricted in particular, an effective amount thereof can be selected on considering a production effiency of reactor, a recovery amount of the used amine and so forth.

An operation of the first step for concentrating the reaction mixture with dehydration until a concentration of the bismethohalide to water reaches not less than 85% by weight finds its importance in that the operation is performed at a temperature of not more than 130°C, preferably not more than 120°C.

In case the above dehydration operation is carried out at a temperature beyond 130°C, it causes a remarkable drop of the yield, which means an extreme disadvantage on aspect of the yield. The reaction mixture should be concentrated with dehydration at a temperature of not more than 130°C until a concentration of the bismethohalide to water reaches preferably 90% by weight, which causes further improvement on the yield of the etheramine and hence more advantage on aspect of the yield. The operation of removing water with distillation as above can be usually performed under the ordinary pressure or reduced pressure, and the performance under the reduced pressure renders effective dehydration feasible, which is preferable, but makes no restriction in particular. The dehydration is usually completed by the operation of 0.5—10 hours under the pressure of 1—750 mmHg, and the dehydration operation of 0.5—5 hours under 50—300 mmHg, which is preferable, renders more effective operation feasible and further can give favorable effect to the yield of the etheramine. The reaction mixture obtained by performing the first step operation forms a heterogeneous reaction mixture consisting of a solid-liquid phase in which a part of the bismethohalide deposits and disperses mainly into the high boiling point aliphatic amine.

The reaction of the second step is featured by heating the above reaction mixture at a temperature of not lower than 140°C, preferably not lower than 160°C to perform a heterogeneous reaction.

The reaction of the bismethohalide with the high boiling point aliphatic amine can proceed usually even at 140°C, but the reaction rate thereof is so slow that it can not be said to be effective on industrial aspect, because it belongs to the heterogeneous reaction.

Therefore, it is preferable to perform the heterogeneous reaction at a temperature of not lower than 160°C, which can form the desired etheramine with high efficiency. Generally speaking, the heterogeneous reaction, in which contacts of solid and liquid are not sufficient in usual cases, can not be said to be advantageous on aspect of the reaction rate. On the contrary, the method of producing the etheramine by this invention can provide the etheramine with a surprisingly high yield, which hence becomes extremely advantageous on industrial aspect.

The heterogeneous reaction can be carried out in any condition of the ordinary pressure, reduced pressure and increased pressure, and there is no particular restriction in the reaction pressure. In the reaction of the ordinary pressure or reduced pressure, the reaction can be carried out with distilling off the resultant etheramine from the reaction mixture. On the other hand, in the reaction of the increased pressure, the etheramine can be recovered by distillation from a resulting homogeneous solution after the reaction is completed.

In order to produce said etheramine industrially, it is necessary to elevate economical efficiency of the process by employing repeatedly the aliphatic amine in the reaction. That is, after the reaction, an alkali compound is added to the reaction mixture comprising hydrochloric acid salt of the amine to liberate the amine, which is recovered.

As to the alkali compound used for the neutralization, there is no restriction in particular, and hydroxide, carbonate and the like of alkali metal can be exemplified therefor. Usually, sodium hydroxide or potassium hydroxide can be used as a solution thereof, and the addition amount thereof is not less than twice moles of the starting bismethohalide, i.e. 2.0—2.2 times moles equivalent. After the neutralization, the aliphatic amine and said etheramine can be recovered through ordinary distillation, filtration, extraction and other separating means. The recovered aliphatic amine can be again in the next reaction repeatedly. The repeated employing of the aliphatic amine as a demethylation agent increases the resulting amount of the etheramine per unit weight of the amine to elevate the economical efficiency of the process remarkably.

As stated above, in case of reacting the starting bismethohalide with the high boiling point amine it renders the production of the etheramine with high efficiency which extremely surpasses the conventional technique feasible to perform the heterogeneous reaction consisting of essentially solid-liquid phase with selecting the reaction processes proposed by this invention. This invention is further explained hereinafter by examples, which do not provide this invention with any restriction.

Example 1

Preparation of bismethochloride of bis[β - (N,N - dimethylamino)ethyl]ether

Into an autoclave of inside volume 5 liter equipped with a magnetic stirrer, 2,820g of 30% trimethylamine aqueous solution (846g of trimethylamine) and 860g of bis(β - chloroethyl)-ether were introduced and heated at 70°C to be subjected to a reaction for 6 hours. After the reaction, excess of unreacted trimethylamine was purged out, and the reaction mixture was analysed. As a result of quantitative analysis of chlorine ion by Volhard method, it was 12.0 equivalents (theoretical value being 12.02 equivalents).

Through $^1H$-NMR and $^{13}C$-NMR analysis of the reaction mixture, it was identified that the product was bismethochloride of bis[β - (N,N - dimethylamino)ethyl]ether and it was produced nearly quantitatively. The total weight of the remained reaction mixture after the unreacted trimethylamine was purged was 3,530g, and the concentration of the bismethochloride of bis[β - (N,N - dimethylamino)ethyl]ether therein was 44.5% by weight.

Preparation of bis[β - (N,N - dimethylamino)-ethyl]ether

Into a glass four-necked flask equipped with a stirrer, thermometer, dropping funnel and short column for distillation of length 30 cm (filled with glass Rashig rings), 292g of triethylenetetramine was added and heated at 120°C. From the dropping funnel, 587g of an aqueous solution having a concentration of 44.5% by weight of bismethochloride of bis[β - (N,N - dimethylamino)ethyl]ether (hereinafter referred to as the bismethochloride) (261.2g of the bismethochloride and 325.8g of water) was added thereinto, and the inside temperature thereof was maintained at 105°C. With keeping the inside pressure 110 mmHg, water was distilled off. At the time 310g of water was distilled off (the concentration of the bismethochloride to water in the reaction mixture was 94%), the temperature of the reaction mixture which formed a heterogeneous solid-liquid phase was raised from 105°C to 170°C. The reaction was carried out for 3.5 hours in a region of substantial reaction temperature of 140°C to 170°C. A distillate, 164.9g, was obtained at a temperature of the column top of 100—130°C (110 mmHg). As a result of gas-chromatographic analysis of this distillate, 140.2g of bis[β - (N,N - dimethylamino)ethyl]ether (hereinafter referred to as the etheramine) was identified. The yield of the etheramine was 87%.

Example 2

In the same glass four-necked flask as in Example 1, 472g of tetraethylenepentamine was added and heated at 110°C. From the dropping funnel, 587g of 44.5% aqueous solution of bis[β - (N,N - dimethylamino)ethyl]ether was added thereinto (which contained 261.2g of the bismethochloride), the inside temperature was kept at 115°C. With maintaining the inside pressure 140 mmHg, a distillate, 297g, was obtained at a temperature of column top of 45—80°C. As a result of gas-chromatographic analysis thereof, the distillate is water at all.

In the reaction mixture consisting of solid-liquid phase at the time 297g of water was distilled off, the concentration of the bismethochloride to water was 90%.

The temperature of reaction mixture was raised from 115°C to 175°C, and the reaction was carried out at a region of substantial reaction temperature of 140°C to 175°C for 4 hours. A distillate, 170g, was obtained at a column top temperature of 110—135°C (140 mmHg). As a result of gas-chromatographic analysis of this distillate it could be identified that 142.6g of the etheramine was present. The yield of the etheramine was 88.5%.

Example 3

Into a stainless autoclave of a magnetic stirrer type, 429g of triethylenetetramine and 587g of 44.5% aqueous solution of bismethochloride of bis[β - (N,N - dimethylamino)ethyl]ether (which contained 261.2g of the bismethochloride) were added, and heated at 90—100°C. With maintaining the inside pressure 150—170 mmHg 300g of water was distilled away through a glass short column installed in a valve on the upper cover of the autovlave over a period of 1 hour. At the time when 300g of water was distilled off, the concentration of the bismethochloride to water in the reaction mixture was 9.1%. After entirely closing the valve of the autoclave and elevating

the inside temperature up to 177°C under the gauge pressure of 1—3 kg/cm², the reaction was carried out for 2.5 hours. After completion of the reacting, total amount of the reaction mixture was transferred in the glass four-necked flask used in Example 1 and was subjected to a crude distillation to give 145g of a distillate at a column top temperature of 115—132°C (140 mmHg). As a result of gas-chromatographic analysis of this distillate, it was identified that 138.6g of the etheramine was present. The yield of the etheramine was 86%.

Comparative Example 1

Into the same glass four-necked flask as in Example 1, 402g of triethylenetetramine was added and heated at 150°C. From a dropping funnel, 587g of 44.5% aqueous solution of bismethochloride of bis[β - (N,N - dimethylamino)-ethyl]ether (which contained 261.2g of the bismethochloride) was added thereinto, and the inside temperature there was kept at 150°C. With maintaining the inside pressure 170 mmHg, water and the reaction product were distilled out. At column top temperatures of 65—130°C and more than 130°C, 346g of a distillate and 134g of another distillate were obtained respectively. As a result of gas-chromatographic analysis of each distillate, it was identified that 109.5g of the etheramine was formed. The yield of the etheramine was 68%.

**Claims**

1. A method for producing bis[β - (N,N - dimethylamino)ethyl]ether wherein an aqueous solution of a bismethohalide of bis[β - (N,N - dimethylamino)ethyl]ether represented by the following general formula:

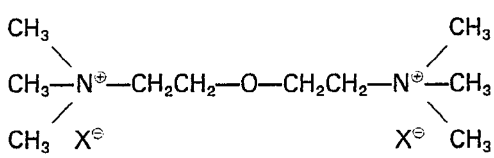

wherein X denotes a halogen atom, is reacted with an aliphatic amine having boiling point of not lower than 220°C and having a primary amino group in the molecule thereof, which is characterized in comprising two steps of:

(a) removing water from the reaction mixture of said bismethohalide aqueous solution and said aliphatic amine with distillation thereof at a temperature of not more than 130°C to render the concentration of said bismethohalide to water to be not lower than 85% by weight, and

(b) performing a heterogeneous reaction in the resulting solid-liquid phase mixture by heating it at a temperature of not lower than 140°C.

2. The method of claim 1, wherein the aliphatic amine is selected from polyethylenepolyamines.

3. The method of claim 1, wherein not less than 2 moles of the aliphatic amine are employed per 1 mol of the bismethohalide.

4. The method of claim 1, wherein step (a), removing water from the reaction mixture with distillation, is carried out under reduced pressure.

**Patentansprüche**

1. Verfahren zur Herstellung von Bis[β - (N,N - dimethylamino)ethyl]ether durch Umsetzen einer wässrigen Lösung eines Bismethohalogenids eines Bis - [β - (N,N - dimethylamino)-ethyl]-ethers der allgemeinen Formel

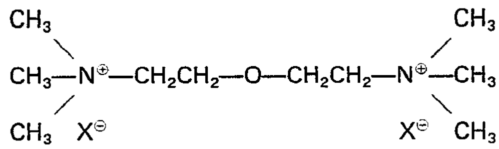

in der für ein Halogenatom steht, mit einem aliphatischen Amin, welches einern Siedepunkt von nicht weniger als 220°C und eine primäre Aminogruppe in seinem Molekül aufweist, dadurch gekennzeichnet, daß es die beiden folgenden Schritte aufweist:

(a) Abtrennen von Wasser aus der Mischung ds Reaktion der wässrigen Lösung des Bismethohalogenids mit dem aliphatischen Amin durch Destillation bei einer Temperatur von nicht mehr als 130°C bis zu einer Konzentration des Bismethohalogenids in Wasser von nicht weniger als 85 Gew.-% und

(b) Durchführen einer heterogenen Reaktion in der erhaltenen Feststoff - Flüssigkeits - Phasenmischung durch Erhitzen auf eine Temperatur von nicht weniger als 140°C.

2. Verfahren nach Anspruch 1, dadurch gekennzeichnet, daß das aliphatische Amin aus Polyethylenpolyaminen ausgewählt wird.

3. Verfahren nach Anspruch 1, dadurch gekennzeichnet, daß nicht weniger als 2 Mol des aliphatischen Amins pro 1 Mol des Bismethohalogenids verwendet werden.

4. Verfahren nach Anspruch 1, dadurch gekennzeichnet, daß die Stufe (a) der Abtrennung des Wassers aus der Reaktionsmischung durch Destillation unter vermindertem Druck durchgeführt wird.

**Revendications**

1. Procédé pour la préparation de bis[β - (N,N - diméthylamino)éthyl]éther, dans lequel on fait réagir une solution aqueuse d'un bisméthohalogénure de bis[β - (N,N - diméthylamino)-éthyl]éther représenté par la formule générale:

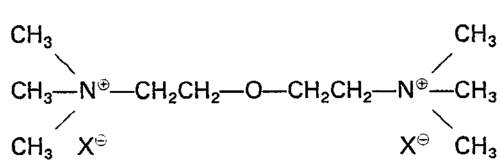

dans laquelle X représente un halogène, avec une amine aliphatique ayant un point d'ébullition d'au

moins 220°C et possédant un groupe amino primaire dans sa molécule, caractérisé en ce qu'il comprend les deux e]apes de

(a) élimination de l'eau hors du mélange réactionnel constitué de la solution aqueuse dudit bisméthо-halogénure et de ladite amine aliphatique, par distillation de celui-ci à une température ne dépassant pas 130°C, pour amener la concentration dudit bisméthо-halogénure par rapport à l'eau à 85% en poids au moins, et

(b) mise en oeuvre d'une réaction hétérogène dans le mélange de phases liquide-solide résultant, par chauffage de celui-ci à une température d'au moins 140°C.

2. Procédé selon la revendication 1, dans lequel l'amine aliphatique est choisie parmi les polyéthylènepolyamines.

3. Procédé selon la revendication 1, dans lequel on utilise au moins 2 moles de l'amine aliphatique par mole du bisméthо-halogénure.

4. Procédé selon la revendication 1, dans lequel l'étape (a) d'élimination d'eau hors du mélange réactionnel par distillation est effectuée sous pression réduite.